Europäisches Patentamt

European Patent Office

Office européen des brevets

(19)

(11) Veröffentlichungsnummer: **0 200 226**

**A1**

(12) **EUROPÄISCHE PATENTANMELDUNG**

(21) Anmeldenummer: **86105998.8**

(22) Anmeldetag: **01.05.86**

(51) Int. Cl.⁴: **C 12 Q 1/18**
**C 12 M 1/12**
**//C12M1/04, (C12Q1/18,**
**C12R1:36)**

(30) Priorität: **02.05.85 DE 3515753**

(43) Veröffentlichungstag der Anmeldung:
**05.11.86 Patentblatt 86/45**

(84) Benannte Vertragsstaaten:
**AT BE CH DE FR GB IT LI LU NL SE**

(71) Anmelder: **Rosin, Harry, Prof. Dr.**
**Sperberweg 4**
**D-4006 Erkrath 1(DE)**

(71) Anmelder: **Preisendanz, Hans**
**Moltkestrasse 37**
**D-4156 Willich 1(DE)**

(72) Erfinder: **Rosin, Harry, Prof. Dr.**
**Sperberweg 4**
**D-4006 Erkrath 1(DE)**

(72) Erfinder: **Preisendanz, Hans**
**Moltkestrasse 37**
**D-4156 Willich 1(DE)**

(74) Vertreter: **Türk, Gille, Hrabal**
**Bruckner Strasse 20**
**D-4000 Düsseldorf 13(DE)**

(54) Verfahren und Vorrichtung zur Bestimmung der Wirkung von Chemotherapeutika auf das Wachstum von Mikroorganismen und/oder Zellen.

(57) Verfahren und Vorrichtung zur Bestimmung der Wirkung von Chemotherapeutika auf das Wachstum von Mikroorganismen und/oder Zellen, wobei die zu untersuchenden Mikroorganismen und/oder Zellen in ein Chemotherapeutikum enthaltendes Nährmedium inokuliert und darin bebrütet werden und ihr Wachstumsverhalten bestimmt wird, wobei das beimpfte Nährmedium mit einem sterilen, das Chemotherapeutikum ebenfalls enthaltenden Nährmedium derart in Verbindung gebracht wird, daß Nährstoffe, Chemotherapeutikum und, falls gewünscht, Stoffwechselprodukte der Mikroorganismen und/oder Zellen, nicht jedoch die Mikroorganismen selbst, zwischen den Nährmedien permeiren.

Patentanmelder:

Dr. med. Harry Rosin

Sperberweg 4

4006 Erkrath 1


Hans Preisendanz

Moltkestr. 37

4156 Willich 1

Verfahren und Vorrichtung zur Bestimmung der Wirkung von Chemotherapeutika auf das Wachstum von Mikroorganismen und/oder Zellen.


Die vorliegende Erfindung betrifft ein Verfahren zur Bestimmung der Wirkung von Chemotherapeutika auf das Wachstum von
Mikroorganismen und/oder Zellen, wobei die zu untersuchenden
Mikroorganismen und/oder Zellen in ein das Chemotherapeutikum
enthaltendes Nährmedium inokuliert und darin bebrütet werden
und ihr Wachstumsverhalten bestimmt wird,
d a d u r c h   g e k e n n z e i c h n e t, daß das beimpfte
Nährmedium mit einem sterilen, das Chemotherapeutikum ebenfalls
enthaltenden, Nährmedium derart in Verbindung gebracht wird,
daß die Nährstoffe, Chemotherapeutikum und, falls gewünscht,
Stoffwechselprodukte der Mikroorganismen und/oder Zellen, nicht
jedoch die Mikroorganismen und/oder Zellen selbst, zwischen
den Nährmedien permeiren.
Unter dem Begriff Wirkung wird der Einfluß von äußeren Faktoren
auf das Wachstumsverhalten von Mikroorganismen und/oder Zellen
verstanden. Insbesondere kennzeichnet der Begriff die Hemmwirkung eines Chemotherapeutikums auf das Wachstumsverhalten
von Mikroorganismen beispielsweise eine wachstumsverlangsamende,
aufhebende oder abtötende Wirkung. Er kann aber auch als die
wachstumsfördernde Wirkung eines Wirkstoffes auf die Kulturen
von Mikroorganismen und/oder Zellen verstanden werden.
Unter Chemotherapeutikum werden Wirkstoffe synthetischen, teilsynthetischen oder natürlichen Ursprungs verstanden, die von
medizinischem und/oder mikrobiologischem Interesse sind.

Insbesondere bezeichnet der Begriff Chemotherapeutikum antimikrobiell wirkende Stoffe. Dazu gehören auch die Antibiotika. Unter Chemotherapeutikum wird auch eine Kombination mehrerer Wirkstoffe verstanden.

Das Verfahren ist auf Mikroorganismen von medizinischem und/oder mikrobiologischem Interesse anwendbar. Dazu gehören mikrobielle, insbesondere bakterielle Krankheitserreger. Das Verfahren ist auch auf Zellen von medizinischem und/oder mikrobiologischem Interesse anwendbar, sowie auf Kombinationen von Mikroorganismen und Zellen.

Das Verfahren zur Bestimmung der Wirkung von Chemotherapeutika betrifft dementsprechend eine Methode zur Untersuchung des Wachstumsverhaltens von Mikroorganismen und/oder Zellen in Gegenwart von Chemotherapeutika, insbesondere zur Prüfung der Empfindlichkeit von Mikroorganismen und/oder Zellen gegen Chemotherapeutika. Das Verfahren kann zur Bestimmung der antimikrobiellen Aktivität von Chemotherapeutika eingesetzt werden. Bevorzugt dient das Verfahren der Bestimmung der minimalen Hemmkonzentration (MHK) und der minimalen bakteriziden Konzentration (MBK) von Chemotherapeutika gegenüber bakteriellen Krankheitserregern. Weiterhin kann das Verfahren in mikrobiologischen Laboratorien zur Bestimmung der Häufigkeit von resistenten Mutanten, zur Bestimmung der Resistenzentwicklung oder zur Untersuchung von Kulturbedingungen von Mikroorganismen, die oder deren Produkte von wirtschaftlichem Interesse sind, eingesetzt werden.

Es sind mehrere Verfahren zur Bestimmung der Hemmwirkung von Chemotherapeutika auf das Wachstum von Mikroorganismen bekannt. Beispielsweise werden vom Deutschen Institut für Normung (DIN) die folgenden Verfahren zur Bestimmung der minimalen Hemmkonzentration (MHK) und der minimalen bakteriziden Konzentration (MBK) empfohlen.

Beim Agardiffusionstest ergeben sich durch die Diffusion des Wirkstoffes auf beimpften festen Nährmedien wachstumsfreie Hemmhöfe. Unter definierten Bedingungen sind die Durchmesser der Hemmhöfe den MHK-Werten der Wirkstoffe für den untersuchten Erreger korrelierbar.

Bei der Bouillonverdünnungsmethode werden mehrere Röhrchen mit gleichen Volumina einer beimpften Bouillon und darin gelöstem Wirkstoff bei schrittweise (geometrisch oder arithmetrisch) abnehmender Wirkstoffkonzentration beschickt. Die niedrigste Konzentration die ein makroskopisch sichtbares Wachstum des Inokulums verhindert, ist die minimale Hemmkonzentration (MHK). Pro Reagenzglasröhrchen ist bei dieser Makro- Bouillonverdünnungsmethode nach DIN 58940 T5 ein Ansatzvolumen von 2 ml enthalten.

Bei der Agar-Verdünnungsmethode wird dieses Verfahren zur Feststellung der MHK-Werte analog auf ein Agarmedium mit abgestuften Wirkstoffkonzentrationen angewandt.

Ein Mikrobouillondilutionstest wird seit kurzem kommerziell angeboten. Es handelt sich hierbei um einen im Mikromaßstab durchzuführenden Test nach der Bouillonverdünnungsmethode, wobei rechteckige Kunststoffplatten mit einer geeigneten Anzahl von Vertiefungen mit jeweils mindestens 100 µl Fassungsvermögen mit der beimpften Bouillon und darin gelöstem Wirkstoff bei schrittweise abnehmender Wirkstoffkonzentration beschickt werden. Auch hier wird der MHK-Wert direkt ermittelt.

Bei diesen Bestimmungsverfahren sind die darin angewandten Bedingungen, besonders die Ausgangsbedingungen, z.B. die Nährmedien, aus technischen bzw. qualitativen Gründen der Reproduzierbarkeit so einfach wie möglich gehalten. Dies bedeutet aber, daß die Verhältnisse in vitro von denen in vivo stark abweichen, die so gewonnenen Ergebnisse sind in hohem Maße methodischen Variationen unterlegen und nur bedingt auf das Infektionsgeschehen im lebenden Organismus übertragbar.

Trotz der großen Diskrepanz zwischen den Verhältnissen in vivo und in vitro wird versucht, empirisch ermittelte Verfahrensbedingungen in vitro anzuwenden, die eine Voraussage auf das Therapieergebnis in vivo zulassen. Dazu werden bei Testbeginn bestimmte Verfahrensbedingungen vorgegeben, z.B. Wirkstoffkonzentrationen, Nährstoffzusammensetzung (soweit bei biologischem Material möglich), pH-Wert, Inkubationsmilieu (den Wachstumsansprüchen der Mikroorganismen angepasst), Höhe des Inokulums usw..

Diese Bedingungen werden jedoch nicht über die gesamte Testdauer konstant beibehalten, da sich häufig während der Bebrütung durch Wachstum oder Untergang der Keime und/oder Degradation der eingesetzten Substanzen starke Veränderungen ergeben.

So bleibt die verfügbare Konzentration des Wirkstoffes hinter der vorgegebenen Konzentration zurück, dadurch, daß der Wirkstoff unkontrolliert an die Mikroorganismen, einen Teil ihrer Stoffwechselprodukte, an Bestandteile des Nährmediums und/oder an das Material des Testgefäßes chemo-absorbtiv gebunden wird.

Während der Inkubation tritt weiterhin durch wachsende Keime eine zunehmende Erschöpfung des Nährmediums ein, da in geschlossenen Systemen gearbeitet wird. Das Wachstum, insbesondere die Wachstumsgeschwindigkeit der Mikroorganismen, hängt aber entscheidend von einem ausreichenden Nahrungsangebot ab.

Vorteilhaft für das Wachstumsverhalten der Mikroorganismen - und in manchen Fällen auch für die chemische Stabilität des Wirkstoffs - ist eine Konstanz des pH-Wertes. Viele Mikroorganismen zeigen ein optimales Wachstumsverhalten nur in einem engbegrenzten pH-Bereich. Der pH-Wert des Testmediums kann sich aber während der Inkubation unter dem Einfluss von Stoffwechselprodukten signifikant verändern mit der Folge einer Verschlechterung der Wachstumsbedingungen für einen empfindlichen Mikroorganismus oder einer Reduktion der Aktivität des Chemotherapeutikums. Weiterhin können manche der Stoffwechselprodukte aus wachsenden oder zugrundegegangenen Keimen das Wachstum der Kultur inhibieren. Schließlich können durch Stoffwechsel- und Zersetzungsprodukte, Gaspartialdrücke verändert werden, die ebenfalls wichtig für das Wachstumsverhalten der Mikroorganismen sind.

Eine Quantifizierung dieser Effekte ist kaum möglich, zumal sich ihre Auswirkungen überlagern.

Aufgabe der vorliegenden Erfindung ist daher, ein Bestimmungsverfahren bereitzustellen, mit dem der Wirkstoffeffekt des Chemotherapeutikums unter weitgehendem Ausschluß von Nebenwirkungen (toxische Stoffwechselprodukte und/oder Erschöpfung des Nährmediums) im Verlaufe der Inkubation zu quantifizieren ist. Das Verfahren ermöglicht es weiterhin den pH-Wert und die Gaspartialdrücke im Nährmedium während der Inkubation auf bestimmte Werte einzustellen. Unter Einstellen wird verstanden, daß je nach Untersuchungsziel ein pH-Wert und/oder die Gaspartialdrücke vorgegeben, gezielt variiert oder weitgehend konstant gehalten werden. Schließlich ist mit diesem Verfahren insbesondere auch eine Quantifizierung des freien, ungebundenen und damit antimikrobiell aktiven Anteils des eingesetzten Chemotherapeutikums unter den gewählten Untersuchungsbedingungen bei Bedarf möglich.Gegenstand der Erfindung ist dementsprechend ein Verfahren der eingangs genannten Art, das dadurch gekennzeichnet ist, daß während der Bebrütung beimpftes Nährmedium mit sterilem, das Chemotherapeutikum ebenfalls enthaltenden Nährmedium, derart in Verbindung gebracht wird, daß die Nährstoffe, das Chemotherapeutikum und, falls gewünscht in abstufbarem Grade, Stoffwechselprodukte der Mikroorganismen und/oder Zellen, nicht jedoch die Mikroorganismen und/oder Zellen selbst, zwischen den Nährmedien permeiren.

Gemäß einer bevorzugten Ausführungsform wird gleichzeitig das beimpfte oder das sterile Nährmedium mit einem inerten Medium derart in Verbindung gebracht, daß freie, ungebundene Moleküle des Chemotherapeutikums in das inerte Medium diffundieren. Die sich einstellende Konzentration dient als Maß für die aktive Konzentration des Chemotherapeutikums im Nährmedium. Die Abgrenzung der verschiedenen Medien ist dadurch gekennzeichnet, daß Nährstoffe, Chemotherapeutikum und, falls gewünscht, Stoffwechselprodukte der Mikroorganismen durch eine oder mehrere semipermeable Wände zwischen den Nährmedien permeiren und das Chemotherapeutikum durch eine oder mehrere semipermeable Wände in das inerte Medium diffundiert.

0200226

Im Folgenden wird die Abgrenzung des beimpften Nährmediums Kulturkammer genannt, die Abgrenzung des sterilen Nährmediums heißt Reservoirkammer und die Ab- grenzung des inerten wässrigen bzw. nicht-wässrigen Mediums heißt Testkammer.

Durch vorangehende Bestimmungen des freien, von den Bestandteilen des Nährmediums nicht gebundenen Anteils an der jeweils durch Einwaage hergestellten Gesamtkonzentration des Chemotherapeutikums wird mit dem hier beschriebenen Verfahren jede gewünschte freie Wirkstoffkonzentration reproduzierbar in jedwedem Nährmedium einstellbar.

Als Nährmedien können in dem Verfahren übliche flüssige Nährmedien eingesetzt werden. Es versteht sich von selbst, daß das nicht beimpfte Nährmedium steril sein muß. Es können herkömm- liche flüssige Nährmedien, wie z.B. Mueller-Hinton-Medium, brain-heart-Bouillon u.a., eingesetzt werden. Diesen herkömmlichen flüssigen Nährmedien können Zusätze gezielt addiert werden, deren spezieller Einfluß auf das Keimwachstum und/oder die Hemmwirkung und/oder die aktive, frei verfügbare Konzentration des eingesetzten Chemotherapeutikums dann untersucht wird; beispielsweise seien hier Eiweißstoffe genannt, von denen insbesondere Albumin in der Lage ist, Antibiotika zu binden, oder auch Lipide oder lipoprotein- bzw. lipoproteidhaltige Nährstoffe. Verfahrensgemäß können vor· allem Nährstoffe eingesetzt werden, die weitergehend als ·bisher in-vivo-Verhältnissen angepasst sind. Auch Nährmedien, die die Bildung extrazellulärer Polysaccharide und Glycoproteine (Glycocalix-Bildung) fördern, sind einsetzbar. Dem zu beimpfenden Nährmedium können auch weitere Materialien, wie z.B. Eiterproben zugesetzt werden. Die Art des zu verwendenden Nährmediums richtet sich dabei nach dem Zweck der Untersuchung, der Art des zu untersuchenden Mikroorganismus und der Art des zu untersuchenden Chemotherapeutikums. Die freie, ungebundene Konzentration des Chemotherapeutikums läßt sich als sehr wichtiger Parameter seiner Aktivität dennoch stets wie gewünscht einstellen.

Nährmedium und Chemotherapeutikum befinden sich in einer oder mehreren Kulturkammern und Reservoirkammern. Die Konzentration des Chemotherapeutikums im Nährmedium ist dabei dem Zweck der Bestimmung angepaßt und liegt in der Regel im Bereich seiner therapeutischen Breite oder im Bereich $2^7$ bis $2^{-7}$ µg/ml. Bei der Bestimmung von MBK- und MHK-Werten ist sie durch die Wirksamkeit des Chemotherapeutikums sowie seine pharmakologischen und toxikologischen Eigenschaften vorgegeben. Im Extrem wird sie durch die Löslichkeit des Chemotherapeutikums im Nährmedium begrenzt.

Die Inokulation des Mikroorganismus und/oder der Zellen erfolgt in das Nährmedium in die dafür vorgesehene(n) Kulturkammer(n), Dabei wird in der Regel die für die Art der Bestimmung (MHK-, MBK-Wert) erforderliche und übliche Keimzahl bzw. Zellenzahl vorgegeben. Es können aber auch geringere oder größere Keimkonzentrationen angewandt werden, je nach Ziel der Bestimmung. Beispielsweise können zur Bestimmung der in einer Mikroorganismuspopulation zu erwartenden Anzahl von resistenten Mutanten in verschiedene Kulturkammern jeweils verschieden hohe Inokula bei gleicher Konzentration des Chemotherapeutikums bebrütet und untersucht werden.

Die Reservoirkammer enthält steriles Nährmedium und Chemotherapeutikum. Sie dient weiterhin der Aufnahme der Stoffwechselprodukte der kultivierten Mikroorganismen und/oder Zellen aus der oder den Kulturkammern. Die Kulturkammern und Reservoirkammern sind durch eine oder mehrere semipermeable Wände von einander getrennt, die für die zu kultivierenden Mikroorganismen und/oder Zellen und, wenn gewünscht, einen Teil der Stoffwechselprodukte undurchlässig sind. Im allgemeinen liegt der Porendurchmesser bei 0,5 nm (entsprechend einer nominalen Molekulargewichtsgrenze NMGG von ca. 500 Dalton) bis 5 µm, bevorzugt bei 5 nm (entsprechend einer NMGG von 5000 Dalton) bis 1 µm, besonders bevorzugt bei 5 nm bis 0,2 µm.

Durch die Wahl der Porengrößen können Verhältnisse geschaffen werden, wie sie in einem mehr geschlossenen System mit langsamem Sekretstrom vorliegen, z.B. in vivo in einem Abszess, oder wie sie in einem mehr offenen System mit höherer Flußrate vorliegen, z.B. in vivo im Liquor cerebrospinalis bei Hirnhautentzündung. Die Bewertung der antimikrobiellen Aktivität von Chemotherapeutika wird auch auf diese Weise der in-vivo-Situation näher angepaßt.

Um ein weitgehend gleichbleibendes Nährstoffangebot, eine gleichbleibende Konzentration an Chemotherapeutikum und eine hinreichende Verdünnung toxischer Stoffwechselprodukte zu gewährleisten, wird das Volumen der Reservoirkammer bevorzugt größer gewählt, als das Volumen aller über semipermeable Wände damit in Verbindung stehenden Kulturkammern zusammen.

Unter weitgehend gleichbleibendem Nährstoffangebot wird verstanden, daß das Volumen der Reservoirkammer groß genug ist, um den Nährstoffverlust in den Kulturkammern durch Verbrauch der Mikroorganismen ohne nennenswerten Konzentrationsverlust auszugleichen. Unter hinreichender Verdünnung der Stoffwechselprodukte wird verstanden, daß das Volumen der Reservoirkammer groß genug ist, die toxischen Stoffwechselprodukte, der in den Kulturkammern kultivierten Mikroorganismen, auf ein für das Wachstum dieser Mikroorganismen unerhebliches Maß zu verdünnen.

Verfahrensgemäß werden die angelegten Kulturen in den Kulturkammern über einen üblichen bzw. den notwendigen Zeitraum bebrütet, d.h. bei schnell wachsenden Aerobiern 16 bis 20 Stunden, bei Anaerobiern in der Regel 48 Stunden usw.

Die Bestimmung des Wachstumsverhaltens erfolgt in gewöhnlicher Weise durch makroskopische Beurteilung des Inhaltes der Kulturkammern oder durch apparative Auswertung der Probe.

Parallel zur Bestimmung der Hemmwirkung von Chemotherapeutika kann in einer angeschlossenen Testkammer der ungebundene, aktive Wirkstoffanteil der vorgegebenen Gesamtkonzentration des Chemotherapeutikums bestimmt werden. Diese Testkammer enthält als inertes Medium ein das Chemotherapeutikum nicht inaktivierendes Lösungsmittel, beispielsweise einen Phosphatpuffer. Das inerte Medium weist vorzugsweise die gleiche Osmolarität, den gleichen pH-Wert und den gleichen kolloidosmotischen Druck auf, wie das Nährmedium in den anderen Kammern. Dabei kann der kolloidosmotische Druck einmal durch geeignete Wahl von Additiven zum inerten Medium eingestellt werden, zum anderen aber auch durch Druckerhöhung in der Testkammer ausgeglichen werden. Unter Additiven werden Substanzen verstanden, die dem inerten Medium beigegeben werden, die nicht aus der Testkammer austreten können und sich gegeüber dem Chemotherapeutikum inert verhalten. In den Testkammern werden wässrige Medien eingesetzt oder auch lipophile Medien verwandt, in dem sich das Chemotherapeutikum gut löst und gegebenenfalls anreichern läßt.

Das inerte Medium in der Testkammmer steht über eine semipermeable Wand mit dem Nährmedium in der Reservoirkammer oder einer Kulturkammer in Verbindung. Diese semipermeable Wand ist durchlässig für das einzusetzende Chemotherapeutikum und für niedermolekulare Anteile im Nährmedium. Sie ist undurchlässig für die Mikroorganismen, die Stoffwechselprodukte der Mikroorganismen und für hochmolekulare Anteile, beispielsweise Eiweißstoffe, im Nährmedium. Im allgemeinen reicht dafür eine Durchlässigkeit von 200 bis 10000 Dalton, bevorzugt von 500 bis 7000 Dalton aus; besonders bevorzugt ist eine Durchlässigkeit von 1000 bis 5000 Dalton, ein Bereich, der auch den Makroliden den Durchtritt gestattet.

In der Testkammer wird mit den üblichen, etwa mikrobiologischen, immunologischen oder HPLC-Verfahren,die Menge an eingewandertem Wirkstoff bestimmt. Diese Wirkstoffmenge ist von der frei verfügbaren Wirkstoffkonzentration im Nährmedium abhängig. Mit Hilfe der Testkammer kann in verschiedenen Medien nach Einstellung des Gleichgewichtes die frei verfügbare Wirkstoffkonzentration im Nährmedium einfach ermittelt werden. Bei Kenntnis der physikochemischen Membraneigenschaften kann die freie Konzentration des Chemotherapeutikums auch vor Erreichen des Gleichgewichtes extrapoliert werden. Um zuverlässige Werte zu erhalten, ist aber Voraussetzung, daß der Inhalt in der Testkammer gegenüber dem Chemotherapeutikum inert ist.

Unter frei verfügbarer Wirkstoffkonzentration wird die Konzentration an Chemotherapeutikum im Nährmedium verstanden, die direkt für die Reaktion mit den Mikroorganismen zur Verfügung steht. Sie unterscheidet sich von der vorgegebenen, durch Einwaage hergestellten, Wirkstoffkonzentration dadurch, daß mehr oder weniger große Konzentrationsanteile an Bestandteile des Nährmediums und Stoffwechselprodukte gebunden und durch Reaktion mit Mikroorganismen absorbiert bzw. inaktiviert werden.

Es ist möglich, in der Testkammer ein lipophiles Medium zu verwenden, in dem sich das Chemotherapeutikum anreichert. Auch in diesem Falle kann aus der Wirkstoffmenge in der Testkammer die frei verfügbare Wirkstoffkonzentration im Nährmedium errechnet werden. Diese Variante ist besonders vorteilhaft bei sehr geringen freien Wirkstoffanteilen.

Die Menge an in die Testkammer eingewandertem Chemotherapeutikum wird vorzugsweise nach Einstellung des Gleichgewichtes bestimmt, d.h. bei Vorliegen der Gleichgewichtskonzentration des Chemotherapeutikums in der Testkammer. Die zur Einstellung des Gleichgewichtes erforderliche Zeit hängt von der verwendeten Membran ab, wird in üblicher Weise empirisch bestimmt und beträgt im allgemeinen 2 bis 10 Stunden. Sie ist im allgemeinen geringer als die Inkubationszeit der Kulturen, so daß die Bestimmung der Hemmwirkung und der freien Wirkstoffkonzentration des Chemotherapeutikums zur gleichen Zeit vorgenommen werden können. Es ist aber auch möglich, die freie Wirkstoffkonzentration aus vor Einstellung der Gleichgewichtskonzentration in der Testkammer erhaltenen Daten mit Hilfe von Tabellen hochzurechnen oder mit Diagrammen zu ermitteln. Voraussetzung sind aber eine standardisierte Testkammer, Membran, und inertes Medium, auch ist es sehr zweckmäßig die anderen Verfahrensbedingungen zu standardisieren, z.B. Temperatur, pH-Wert, Gasströme usw.

Die für ein Chemotherapeutikum in einem Nährmedium ermittelten freien Wirkstoffkonzentrationen können tabellarisch festgehalten werden. Damit ist es möglich, die einmal ermittelten Werte in zukünftigen Bestimmungen wieder zu verwenden. Bei bekannter freier Wirkstoffkonzentration eines Chemotherapeutikums kann also auf die Testkammer verzichtet werden.

Bevorzugt werden die Nährmedien in Kultur- und Reservoirkammern und, falls vorhanden, das inerte Medium, jedes für sich, durchmischt, um eine Beschleunigung der Permeation und Diffusion zu erreichen. In den Kammern werden durch Einleiten oder Kontakt von Gasen die für das Wachstum der Mikroorganismen gewünschten Gaspartialdrücke eingestellt. Die Gase können über Düsen, Fritten oder gasdurchlässige Membranen in jedem Mischungsverhältnis zugeführt werden. Für das Wachstum der Mikroorganismen wichtige Gase sind beispielsweise Sauerstoff, Stickstoff und Kohlendioxid, in bestimmten Fällen aber auch Methan oder Ammoniak. Es versteht sich von selbst, daß der eingeleitete Gasstrom steril filtriert ist. Die Einleitung der Gase erfolgt vorzugsweise in alle Kammern. Dabei ist die Position und Form der Gasdüsen oder der Fritten so gewählt, daß die Kammerinhalte, jede für sich, durch das strömende Gas durchmischt werden. Hierdurch wird die Konvektion beschleunigt und eine schnellere Verteilung der durch die semipermeablen Wände permeirenden oder diffundierenden Stoffe erreicht.

Die Durchmischung der Kammerinhalte kann aber auch durch andere oder zusätzliche Maßnahmen, z.B. durch Rühren oder Schütteln erfolgen.

In allen Kammern wird bevorzugt der pH-Wert auf einen gewünschten Wert eingestellt. Dieser pH-Wert ist in der Regel ein Wert, der für das Wachstum der Mikroorganismen und/oder Zellen, bzw. für die Wirkung des Chemotherapeutikums vorteilhaft oder zuträglich ist oder auch ein Wert, der durch das Untersuchungsziel vorgegeben ist. Im allgemeinen handelt es sich hierbei um pH-Werte im physiologischen Bereich.

Die Einstellung der pH-Werte kann mit Hilfe von Pufferlösungen in üblicher Weise erfolgen. Es ist aber auch möglich und zweckmäßig, den pH-Wert in den Kammern über die Gaspartialdrücke einzustellen und zu steuern. Hierzu wird insbesondere der

$CO_2$-Partialdruck verwandt. Dadurch kann der pH-Wert während der Inkubation in einfacher Weise in engen Grenzen konstant gehalten oder gezielt variiert werden. So hat das erfindungsgemäße Verfahren gegenüber den bekannten Verfahren mehrere Vorteile:

1. Verdünnung toxischer Stoffwechselprodukte durch deren Permeation aus den Kulturkammern in die sterilen Reservoirkammern.

2. Vermeidung einer Erschöpfung des Nährmediums durch Ersatz von Nährstoffen aus den Reservoirkammern.

3. Gezielte Steuerung der Untersuchungsbedingungen, wie z.B. pH-Wert oder Gaspartialdrücke.

4. Erfassung der freien, ungebundenen Konzentrationen der untersuchten Chemotherapeutika in jedweden Nährmedien.

Umgekehrt kann durch entsprechende Anpassung der Gesamtkonzentration des Chemotherapeutikums der Einfluß der verschiedenen Nährmedien auf das Wachstumsverhalten (Resistenz) der Mikroorganismen und/oder Zellen bei gleichem, freiem, ungebundenem Wirkstoffanteil bestimmt werden. Besonders wichtige Perspektiven werden eröffnet, wenn unter Anwendung eines für Mikroben und Leukozyten gleichermaßen geeigneten Nährmilieus die zu untersuchenden Mikroorganismen und Leukozyten in die Kulturkammern inokuliert werden und nun die Phagozytoseleistung bei definierter freier Konzentration des Chemotherapeutikums erfaßbar ist.

Das Verfahren ermöglicht den Vergleich von in in-vitro bestimmten freien, ungebundenen Wirkstoffkonzentrationen mit solchen, die in in-vivo gewonnenen biologischen Flüssigkeiten festgestellt werden. Durch einen solchen Vergleich der in vitro und in vivo bestimmten freien Wirkstoffkonzentrationen sind in-vitro- und in-vivo-Verhältnisse wesentlich besser aneinander angleichbar. Dies ist für therapeutische Empfehlungen von großer Bedeutung.

Das erfindungsgemäße Verfahren kann auf ein statisches System, etwa entsprechend der klassischen Bouillon-Verdünnungsmethode, angewandt werden. Das Verfahren kann aber auch dynamisiert

werden, beispielsweise um die Pharmakokinetik von Chemotherapeutika unter therapeutischen Bedingungen nachzuvollziehen.
Hierzu kann der beschriebene Aufbau mit einem Pumpsystem kombiniert werden, vgl. SANFILIPPO und MORVILLO, Chemotherapy 13,
54 bis 60 (1968); GRASSO et al., Antimicrob. Agents Chemother.,
13, 570 bis 576 (1978). Hierzu kann auch ein Hohlfasersystem
eingesetzt werden, welches durch Dialyse die Antibiotikaspiegel
in den Testkammern verändert. Eine Dynamisierung kann hinsichtlich aller einstellbarer Parameter erfolgen (Wirkstoffkonzentration, pH-Wert, Gaspartialdruck, Temperatur).

Durch geeignete Wahl des Volumens der Reservoirkammer lassen
sich meß- und regeltechnische Eingriffe mit kleinen Hysteresen
bewerkstelligen. Eine Automatisierung des Verfahrens ist ohne
weiteres möglich.

Das erfindungsgemäße Verfahren wird in einer Vorrichtung durchgeführt, dadurch gekennzeichnet, daß es mehrere Kammern, darunter
mindestens eine Kulturkammer für das beimpfte Nährmedium mit
jeweils einer oder mehreren für die zu untersuchenden Mikroorganismen undurchlässigen semipermeablen Wänden und mindestens eine
über die semipermeablen Wand oder Wände damit in Verbindung stehende
Reservoirkammer für das sterile Nährmedium,aufweist.

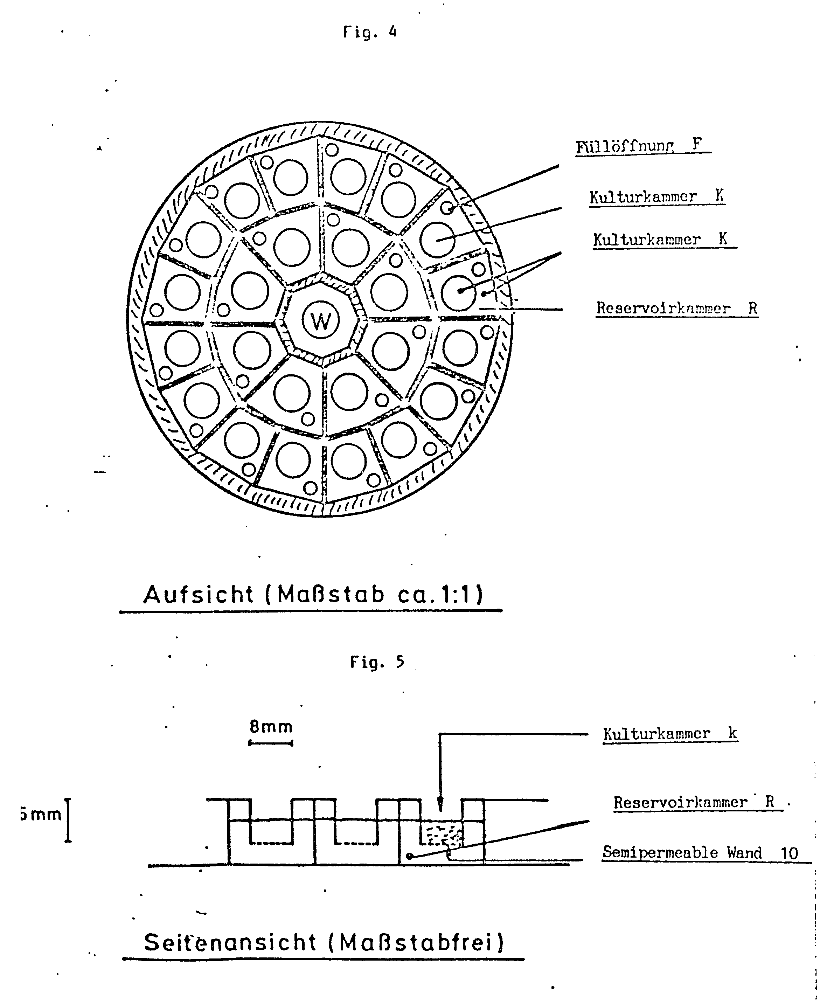

Die Kammern bestehen aus einem gegenüber dem Nährmedium und
dem Chemotherapeutikum beständigen Material. Die Kulturkammern
sind zudem beständig gegenüber den Mikroorganismen. Einer
Reservoirkammer werden je nach Untersuchungsziel eine oder
mehrere Kulturkammern, bei Bedarf auch eine oder mehrere Testkammern, zugeordnet. Eine bevorzugte Ausführungsform (siehe
Beispiel 2) kombiniert eine Reservoirkammer mit 6 Kulturkammern
oder mit 5 Kulturkammern und einer Testkammer. Gemäß einer
anderen bevorzugten Ausführungsform werden mehrere Einheiten
bestehend aus je einer Kulturkammer und je einer Reservoirkammer,
miteinander kombiniert. Diese Kombination kann beispielsweise
die Form und Größe einer herkömmlichen Petrischale mit regelmäßig angeordneten Kulturkammer/Reservoirkammer-Einheiten aufweisen ( Fig. 4 und Fig. 5).

Die Reservoirkammer dient in erster Linie als Puffervolumen zur Verdünnung der Stoffwechselprodukte der Mikroorganismen und/oder Zellen sowie der Aufrechterhaltung einer gleichbleibenden Konzentration an Nährstoff und Chemotherapeutikum. Aus diesem Grunde ist das Volumen der Reservoirkammer bevorzugt größer als das Volumen aller über semipermeable Wände damit in Verbindung stehenden Kulturkammern zusammen. Dabei kann das Verhältnis Gesamtvolumen der Kulturkammern zu Volumen der Reservoirkammer 1:1 bis 1:2000 betragen, bevorzugt 1:5 bis 1:100 und besonders bevorzugt 1:5 bis 1:30.

Die Kulturkammern sind an einer oder mehreren Seiten durch semipermeable Wände abgeschlossen. Diese semipermeablen Wände sind durchlässig für das Chemotherapeutikum, das Nährmedium und, über die Porengröße abstufbar, die von den Mikroorganismen erzeugten Stoffwechselprodukte. Sie sind undurchlässig für die zu untersuchenden Mikroorganismen und/oder Zellen. Der Porendurchmesser richtet sich daher nach der Größe des zu untersuchenden Mikroorganismus und etwaiger zurückzuhaltender Stoffwechselprodukte. Im allgemeinen weisen die semipermeablen Wände zwischen den Kulturkammern und der Reservoirkammer einen Porendurchmesser von 500 Dalton bis 5 µm auf, besonders bevorzugt von 5000 Dalton bis 0,2 µm.

Die semipermeable Wand kann aus Materialien bestehen wie Kunststoffen, z.B. regenerierter Zellulose oder Polycarbonat. Es können aber auch semipermeable Wände auf Basis von entsprechend durchlässigen Metallfilterfolien oder gläsernen oder keramischen Materialien eingesetzt werden.

Die Kammern können im unteren Bereich jeweils einen Gaseinlass aufweisen, beispielsweise in Form einer oder mehrerer Düsen oder Fritten. Die Position und Form der Gaseinlässe ist so gewählt, daß der Gasstrom eine ständige Durchmischung der Kammerinhalte bewirkt. Im oberen Bereich befindet sich dann jeweils ein Gasauslass, der zugleich als Entnahmeöffnung dienen kann.

Die Kammern können Rührvorrichtungen enthalten, mit deren Hilfe die Kammerinhalte, jeweils für sich, zusätzlich zur oder anstelle der Umwälzung durch den Gasstrom durchmischt werden.

In einer weiteren Ausführungsform kann das Mehrkammersystem eine Testkammer mit einem Gaseinlaß im unteren Bereich aufweisen, welche über eine oder mehrere für das Chemotherapeutikum durchlässige semipermeable Wände mit einer der anderen Kammern in Verbindung steht. Für die Testkammer gelten die gleichen Materialanforderungen wie für die Kulturkammern. Die semipermeable Wand ist durchlässig für das Chemotherapeutikum und für niedermolekulare Bestandteile des Nährmediums. Sie ist undurchlässig für hochmolekulare Bestandteile des Nährmediums und für die Mikroorganismen. Sie besitzt eine Durchlässigkeit von 200 bis 100000 Dalton, vorzugsweise von 500 bis 7000 Dalton, besonders bevorzugt von 1000 bis 5000 Dalton.

Die semipermeable Wand kann auch hier aus üblichen Materialien bestehen wie Kunststoffen, z.B. regenerierter Zellulose oder Polycarbonat. Es können aber auch semipermeable Wände auf Basis von entsprechend durchlässigen Metallfilterfolien oder gläsernen oder keramischen Materialien eingesetzt werden.


Die Testkammer steht über eine oder mehrere semipermeable Wände mit der Reservoirkammer oder einer Kulturkammer in Verbindung. Ihr Volumen ist klein im Vergleich zum Volumen der Reservoirkammer. Es kann so groß sein wie das Volumen einer Kulturkammer. Vorzugsweise beträgt das Verhältnis zwischen Testkammervolumen und Kulturkammervolumen 1:1 bis 1:50. Besonders bevorzugt beträgt das Verhältnis 1:1 bis 1:20.

Die Testkammer kann ebenso wie die anderen Kammern einen Gaseinlaß im unteren Bereich, eine Entnahmeöffnung im oberen Bereich sowie eine Rührvorrichtung aufweisen. Die Kammern können so zueinander angeordnet sein, daß die Reservoirkammer die Kulturkammern und gegebenenfalls die Testkammern ganz oder teilweise umschließt. Bei dieser Anordung ist es möglich, eine größere Anzahl von Kulturkammern innerhalb der Reservoirkammer unterzubringen. Die Testkammer kann innerhalb der Reservoirkammer, aber auch innerhalb einer Kulturkammer angeordnet sein.

Gemäß einer bevorzugten Ausführungsform weist die Vorrichtung

eine Kulturkammer pro Reservoirkammer auf, wobei eine regelmäßige Anordnung mehrerer solcher Kulturkammer/Reservoirkammer-Einheiten auf einer Platte, beispielsweise einer Petrischale möglich ist. In einer solchen Einheit kann die Kulturkammer von oben in die Reservoirkammer hineinragen, wobei der Boden der Kulturkammer und/oder die Seitenwände als semipermeable Wand ausgebildet sind. Dabei kann in der Kulturkammer eine optische Hilfe zur Bestimmung des Wachstumsverhaltens der Mikroorganismen und/oder Zellen, als Trübung des Kulturmediums (Verdeutlichung des Tyndall-Effektes), vorhanden sein, beispielsweise in der Form, daß ein Teil der Kammer, insbesondere der Boden verspiegelt, fluoreszierend oder geschwärzt ist. Außer einer entsprechenden Verdeutlichung des Tyndallphänomens kann sinngemäß auch die Änderung eines angelegten elektrischen oder magnetischen Feldes zum Nachweis des Wachstumsverhaltens der Mikroorganismen und/oder Zellen genutzt werden, z.B. unter Verwendung von leitenden Kunststoffen oder hochfrequenten Wechselfeldern. Gemäß dieser bevorzugten Ausführungsform sind in den Kulturkammern und Reservoirkammern bei Bedarf Rührkörper vorhanden, die durch äußere Einwirkung beispielsweise durch Rüttel- oder Schaukelbewegungen, eine Umwälzung der jeweiligen Kammerinhalte bewirken und gleichfalls bei Bedarf als optische Hilfe dienen.

Die Ablesung kann makroskopisch oder bei maschinenkompatibler Formgebung der Kammerpalette, z.B. recheckig, automatisch erfolgen.

In den Abbildungen sind Ausführungsbeispiele der erfindungsgemäßen Vorrichtungen dargestellt, und zwar zeigen

Fig. 1 schematisch ein Dreikammersystem, bestehend aus einer Reservoir-, Kultur- und Testkammer.

Fig. 2 ein Mehrkammersystem, bei dem die Reservoirkammer mehrere Kulturkammereinheiten umgibt.

Fig. 3 eine Kulturkammer und einen Membranhalter aus dem in Fig. 2 dargestellten Mehrkammersystem.

Fig. 4    auf einer Petrischale angeordnete Kulturkammer/
          Reservoirkammer-Einheiten in Draufsicht und

Fig. 5    die Kulturkammer/Reservoirkammer-Einheiten
          aus Fig. 4 in seitlichem Schnitt.

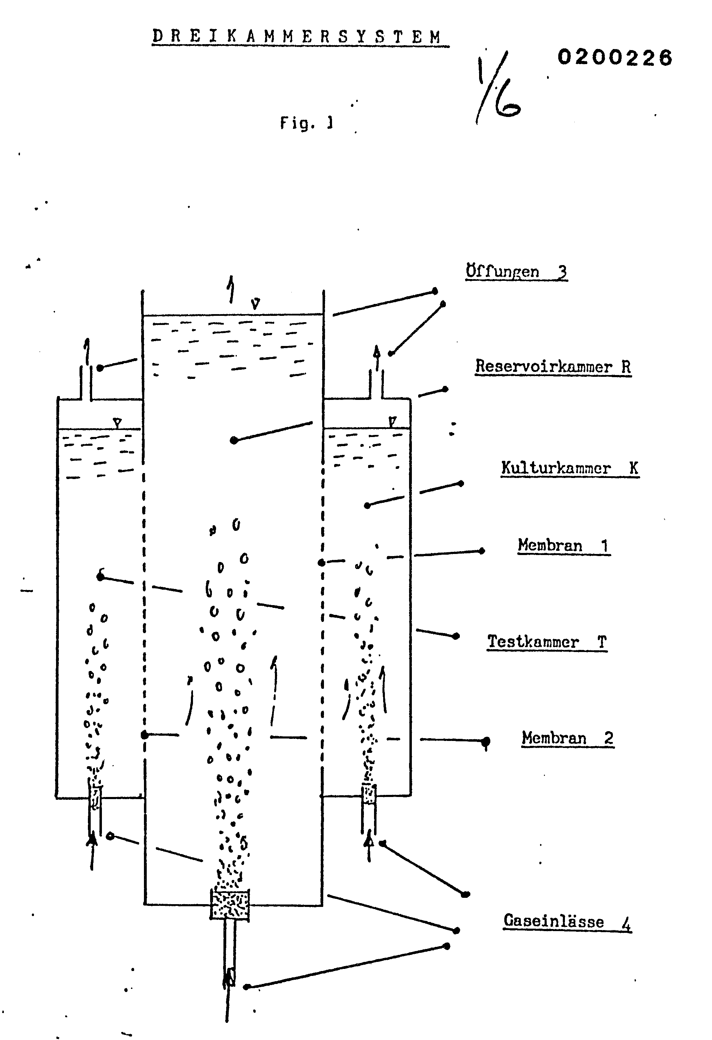

Figur 1 zeigt schematisch ein Dreikammersystem bestehend aus einer Reservoirkammer R für das Nährmedium und Antibiotikum, einer Kulturkammer K für Nährmedium, Antibiotikum und die zu untersuchenden Mikroorganismen und einer Testkammer T für eine Pufferlösung. Die Kammern K und T sind durch semipermeable Membranen 1 und 2 von der Reservoirkammer R getrennt, wobei die Membran 1 für die Mikroorganismen undurchlässig, jedoch für Nährmedium, das Antibiotikum und die Stoffwechselprodukte der Mikroorganismen durchlässig ist. Die Membran 2 zwischen der Testkammer und der Reservoirkammer ist für das Chemotherapeutikum und niedermolekulare Anteile des Nährmediums durchlässig, für Mikroorganismen und hochmolekulare Anteile des Nährmediums undurchlässig. Am oberen Ende der Kammer K und T befinden sich Öffnungen 3, die als Gasaustritte und gleichzeitig der Probennahme dienen. Am Boden aller drei Kammern befinden sich Gaseinlässe 4.

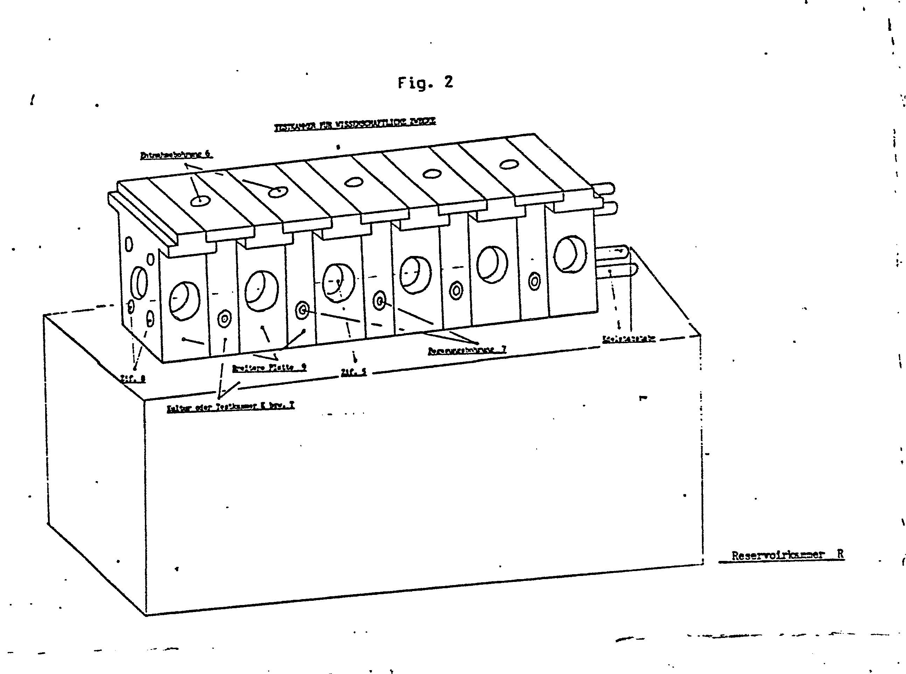

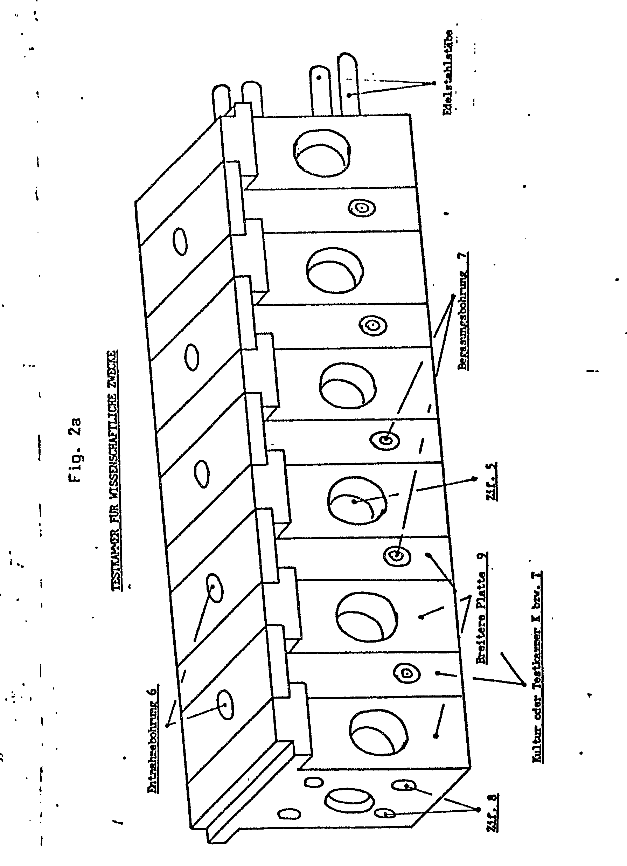

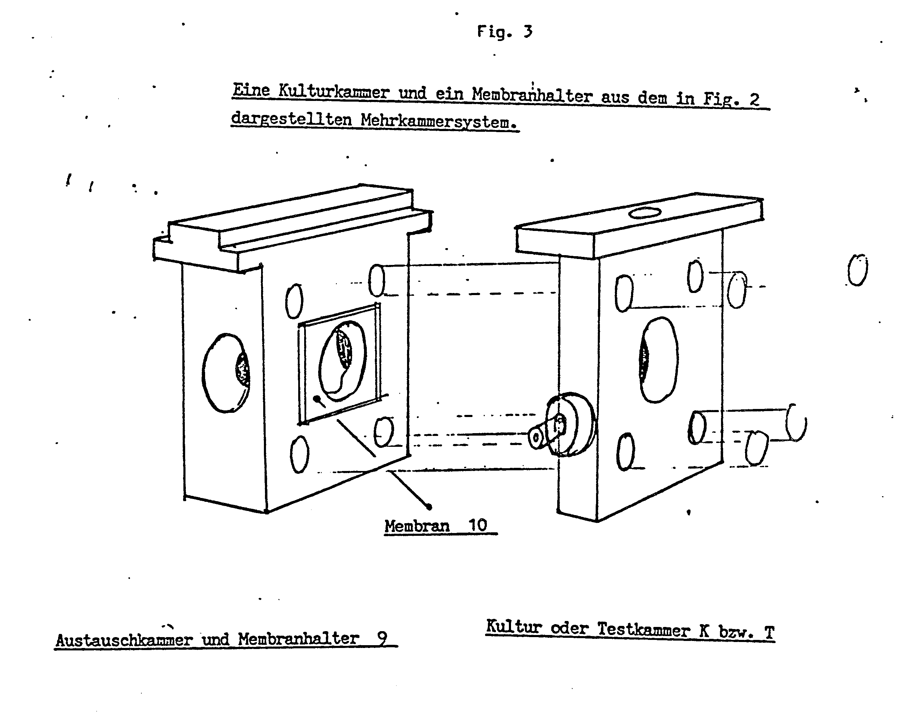

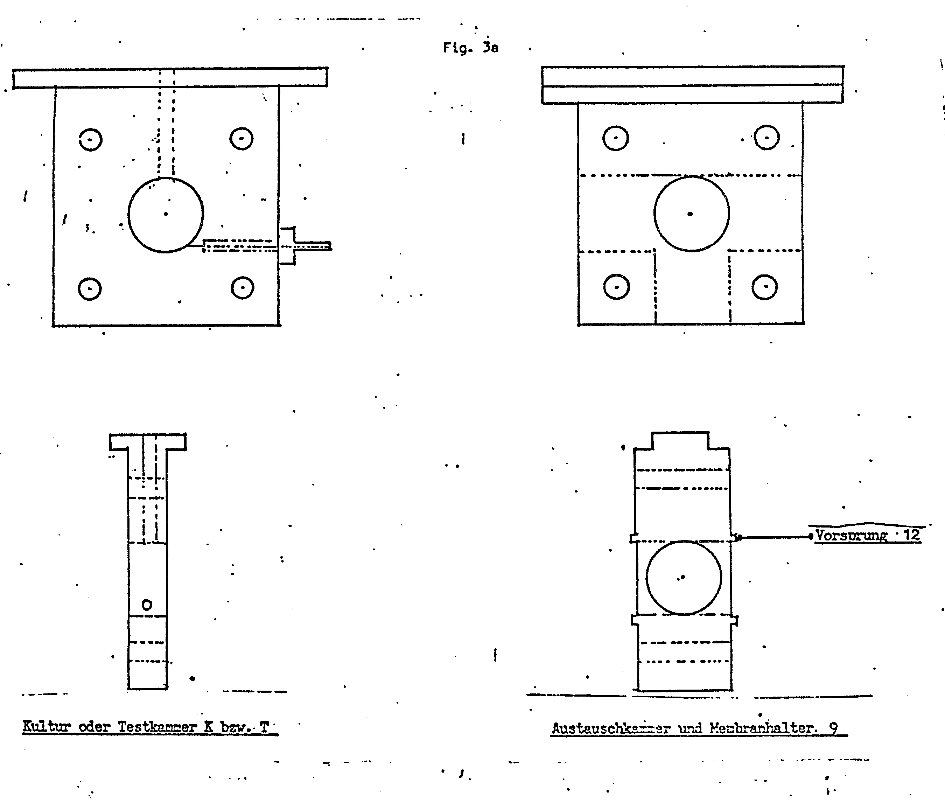

Figur 2 zeigt eine für wissenschaftliche Zwecke geeignete Ausführung der erfindungsgemäßen Vorrichtung bestehend aus einer Reservoirkammer R und mehreren Kulturkammern K in einem Mehrkammersystem. Das Mehrkammersystem besteht aus einer Edelstahlwanne als Reservoirkammer R. In dieser Wanne steht auf einem Polypropylensockel unverschiebbar ein durch 4 Edelstahlstäbe zusammengehaltener Satz von Teflonplatten mit zwei unterschiedlichen Bemaßungen. Die schmaleren Scheiben stellen die Kulturkammern K dar und sind auch als Testkammer T verwendbar. Sie weisen eine Zentralbohrung mit einem Durchmesser von 20 mm, Zif. 5, eine Entnahmebohrung mit einem Durchmesser von 6 mm Zif. 6, eine Begasungsbohrung mit einem Durchmesser von 1 mm, Zif. 7 und vier Führungslöcher für die Edelstahlstähle mit einem Durchmesser von jeweils 8 mm, Zif. 8 auf.

Die breiteren Platten 9 dienen der Abstandshaltung zwischen den Kulturkammern K und als Membranhalter für die semipermeablen Membranen 10, die zwischen den breiten und schmalen Platten eingespannt werden. Sie haben die gleiche zentrale Bohrung Zif. 5 und die gleichen Führungslöcher 8 wie die Kulturkammern K. Darüber hinaus ist diese Zentralbohrung 5 mit gleichgroßen Bohrungen 11 zu den Seiten und nach unten verbunden. An den den benachbarten Kulturkammern K zugewandten Seiten, dient ein ringförmiger Vorsprung 12 um die Zentralbohrung 5 herum als Widerlager und Halterung für die eingespannten semipermeablen Membranen 10.

Die Kulturkammern werden beiderseits von einer semipermeablen Membran aus Polycarbonat (Porengröße 0,1 μm) gegen die Reservoirkammer abgegrenzt. Wird eine der schmalen Teflonscheiben als Testkammer verwandt, wird sie gegen die Reservoirkammer durch eine semipermeable Membran auf Zellulosebasis, z.B. Cuprophan$^R$ (Durchlässigkeit bis zu ca. 10000 Dalton) oder Spectrapor$^R$ (Durchlässigkeit bis zu 3500 Dalton) abgegrenzt.

An die Begasungsbohrung 7 der Kultur- bzw. Testkammmern wird über einen Stutzen ein Begasungsschlauch angeschlossen. Das eingeblasene, steril-filtrierte Gas perlt durch den Inhalt der Kammern. Dabei wird der Inhalt, z.B. Nährmedium, durchmischt und auf einen konstanten Gas- partialdruck sowie pH-Wert gehalten. Das Gas entweicht über die Entnahmeöffnungen 6. Das Nährmedium in der Reservoirkammer wird über ein Hohlfaserbündel, z.B. Cuprophan$^R$ - Hohlfasern, begast.

In die Reservoirwanne wird ein großer ellipsoidförmiger Magnetrührstab eingelegt. Die Reservoirkammer R ist mit 2,5 bis 2,8 1 Nährmedium gefüllt. Der durch die Edelstahlstäbe gehaltene verschraubte Teflonkammer-Satz ist darin eingetaucht. Als Nährmedium kann beispielsweise die kommerziell erhältliche, international empfohlene Mueller-Hinton-Bouillon verwendet werden oder als nährstoffhaltigere Bouillon brain-heart-Bouillon.

Figur 3 zeigt eine Kulturkammer K und einen Membranhalter 9 des oben beschriebenen Mehrkammersystems nach Fig. 2.

Das Mehrkammersystem in Fig. 4 besteht in einer für Routinezwecke bestimmten Ausführung aus einer Palette von Reservoirkammern R mit jeweils einer Kulturkammer K, hier aus durchsichtigem Kunststoff, etwa Polystyrol, zum einmaligen Gebrauch gefertigt. Die Palette mit der äußeren Form einer üblichen Petrischale von 9 cm Durchmesser umfaßt 24 plus 1 Funktionseinheiten, soraß die Wirkung von 12 verschiedenen Therapeutika in jeweils zwei Konzentrationen geprüft werden kann, z.B. in den beiden im Beiblatt 2 zu DIN 58940 Teil 4 aufgeführten Grenzkonzentrationen. In der zentral gelegenen Funktionseinheit W wird das Wachstumsverhalten der zu untersuchenden Mikroorganismen hemmstoffrei geprüft (Wachstumskontrolle).

Die wirkstoffhaltigen Reservoirkammern R haben jeweils gleichen Rauminhalt von ca. 2 ml. Von der Abdeckung her ragen in sie die Kulturkammern K als Kaverne von jeweils ca. 80 $mm^2$ Grundfläche und 8 mm lichter Höhe hinein. Der Boden jeder Kulturkammer besteht aus einer reißfesten semipermeablen Wand 10, im vorgegebenen Beispiel aus Polycarbonat (Porengröße 0,1 µm). Die Kammerpalette wird sterilisiert. Danach wird die Reservoirkammer durch eine kleine Füllöffnung F mit Nährmedium und der gewünschten Menge des jeweils gewählten Chemotherapeutikums unter sterilen Kautelen beschickt. Der freibleibende, nicht gebundene, antimikrobiell wirksame Anteil des Chemotherapeutikums kann durch Vorversuche bestimmt worden sein, z.B. mit der in Abbildung 1 und 2 bereits dargestellten Apparatur. Reservoirkammer und Kulturkammer erhalten denselben Inhalt dergestalt, daß der Flüssigkeitsspiegel in beiden Kammern gleiches Niveau ca. 2 mm unterhalb der Abdeckung einnimmt. Der Inhalt der Kulturkammern beträgt dann 250 µl bei einer Tiefe von 5 mm. Aus Gründen der Praktikabilität und der Stabilität der Chemotherapeutika kann der Inhalt aller Kammern lyophilisiert werden. Vor Gebrauch wird jede Reservoirkammer mit dem durch die Lyophilisation entzogenen Wasservolumen unter sterilen Kautelen aufgefüllt, jede Kulturkammer erhält 150 µl Wasser. Dann werden 100 µl der zu untersuchenden Bakteriensuspension in jede Kulturkammer pipettiert. Die Keimzahl, das Inokulum, in den resultierenden 250 µl Kammerinhalt kann den Empfehlungen in DIN 58940 Teil 5 folgen, d.h. einer Keimzahl von $2 \times 10^5$ bis $2 \times 10^6$ vermehrungsfähigen Keimen je ml entsprechen. Im vorgegebenen Beispiel sind kleine Rührkörper (RK) in jede der Kammern zur Verbesserung der Konvektion eingebracht. Der Rührkörper in der Kulturkammer dient gleichzeitig durch Farbgebung oder Verspiegelung als Hilfe für die makroskopische Beurteilung der mit einer Keimvermehrung eintretenden Trübung. Es besteht auch die Möglichkeit eine entsprechende optische Hilfe an der Membran zu fixieren, z.B. Verspiegelung oder Schwärzung eines Teilbereichs. Wenn die Membraneigenschaften durch die optische Hilfe nicht wesentlich verändert werden, ist die gesamte Membran entsprechend präparierbar. Die semipermeable Wand zwischen Kulturkammer und Reservoirkammer führt zu den beschriebenen Vorteilen des Austausches von Chemotherapeutikum, Stoffwechselprodukten und Nährstoffen zwischen beiden Kammern.

Nach der Inokulation wird das gesamte System - üblicherweise wie in DIN 58940 empfohlen - 16 bis 20 Stunden unter schaukelnden Bewegungen in einem für die zu untersuchenden Mikroorganismen geeigneten Milieu inkubiert, üblicherweise aerob, anaerob oder bei erhöhter $CO_2$-Spannung bei $36 +/- 1^o$ C. Nach der Inkubation erfolgt die makroskopische Ablesung der Untersuchungsergebnisse.

Anstelle einer makroskopischen Ablesung durch den Untersucher ist auch eine maschinelle Ablesung möglich. Die Formgebung der Kammerpalette kann dazu maschinenkompatibel gestaltet werden, z.B. rechteckig. Bei maschineller Ablesung wird u.a. angestrebt, die Inkubationszeit zu verkürzen.

## B E I S P I E L 1

Versuchsdurchführung:

Das Nährmedium: Es werden 2,5 l brain-heart-Bouillon hergestellt, sterilisiert und für den weiteren Gebrauch auf 20° C abgekühlt. Das Nährmedium setzt sich zusammen aus Kalbshirn-Infus 200g, Rinderherz-Infus 250 g, Proteose-Pepton 10 g, Dextrose 2 g, NaCl 5 g und Dinatriumphosphat 2,5 g auf 1000 ml Aqua dest. (pH 7,4 +/- 0,2), zusätzlich angereichert um das Bacto-Supplement B, Fa. Difco, USA.

Das Chemotherapeutikum: Für dieses Beispiel wird Ceftriaxon gewählt, weil es als neues Cephalosporin-Antibiotikum zur Meningitistherapie empfohlen ist und in hohem Prozentsatz an Proteine, Lipide und vor allem an Albumin gebunden wird. Bei einem Nährmediumvolumen von 2,5 l werden 2,5 mg kationen- und hydratwasserfreies Ceftriaxon (entspricht der aktiven Substanz) für die Herstellung einer Gesamtkonzentration von 1 µg/ml Nährlösung benötigt. Die Ausgangssubstanz enthält je nach Charge verschiedene aktive antibakterielle Gewichtsanteile, so daß unter Zuhilfenahme der Herstellerangaben die absolut einzusetzende Menge rechnerisch zu bestimmen ist. Die absolute Menge wird in 5 ml Aqua dest. gelöst und sterilfiltriert, dann der Nährlösung, siehe oben, zugesetzt.

Testkeime: Zur Prüfung der antibakteriellen Aktivität von Ceftriaxon werden als Testkeime 6 Stämme der Spezies Neisseria meningitidis (Meningokokken), häufige Erregern von Hirnhautentzündungen, eingesetzt. Für die Inokulation wird pro Stamm eine Aufschwemmung von 0,5 X $10^6$ Keimen pro ml in 1 ml steriler brain-heart-Bouillon ohne Ceftriaxon hergestellt.

Testvorrichtung: In die Testvorrichtung wird zwischen je einer Kultur- und Austauschkammer eine Polycarbonatmembran (Porendurchmesser 0,1 µm, durchlässig für Nährmedium, Stoffwechselprodukte der Mikroben und für Ceftriaxon) eingespannt. Dieses Kammersystem wird mit einem Rührfisch in die Reservoirkammer eingebracht. Die Gaseinlassdüsen werden mit Silikonschläuchen verbunden. Die gesamte Anlage wird autoklaviert und anschließend auf Raumtemperatur abgekühlt.

Beschicken der Vorrichtung: Es erfolgt unter sterilen Kautelen. Die 6 Keimsuspensionen von je 1 ml werden zusätzlich mit je 1 ml ceftriaxonhaltigem Nährmedium gemischt und in die 6 Kulturkammern gefüllt. Die Reservoirkammer wird mit dem verbliebenem ceftriaxonhaltigem Nährmedium (2494 ml) beschickt. Die Vorrichtung wird auf einen Magnetrührer gestellt, der gleichzeitig die Temperierung übernehmen kann. Ebenfalls kann die Anlage in einen Brutraum gestellt werden. Die Bebrütungstemperatur von 36 +/- 1° C läßt sich auch mit einem Wärmetauscher oder Wasserbad aufrechterhalten. Hier jedoch wird ein Brutraum verwendet. Die Rührvorrichtung (Magnetrührer) und der Gasstrom, z.B. 10% $CO_2$ plus 18% $O_2$ plus 72% $N_2$, verteilen das Antibiotikum gleichmäßig im gesamten Flüssigkeitsvolumen, wobei in den Kulturkammern innerhalb von 30 Minuten der Konzentrationsspiegel des Ceftriaxons sich dem der Reservoirkammer angleicht. Der Verdünnungseffekt durch das 6 ml betragende Volumen der Keimsuspension auf die 2500 ml Nährbouillon ist vernachlässigbar.

Das gesamte System wird 16 bis 20 Stunden lang unter Beibehaltung der Durchmischung mit Hilfe von Rühreinrichtung und Begasung bei ca. 36° C isotherm inkubiert. Diese Inkubationsdauer wird zur Ermittlung der MHK in DIN 58940 T5 empfohlen. Danach wird über die Entnahmebohrung (Gasauslassbohrung) mit einer sterilen Pasteurpipette der Inhalt der Kulturkammern aufgesaugt und in der Pipette makroskopisch nach DIN 58940 begutachtet. Makroskopische Trübung bedeutet nicht ausreichend gehemmtes Wachstum. Im vorliegenden Verfahren wird sichergestellt, daß eine Wachstumshemmung nur die Funktion des Antibiotikumspiegels ist und nicht zusätzlich durch Erschöpfung des Nährmediums oder toxische Stoffwechselprodukte der Keime beeinflußt wird.


B E I S P I E L 2

Vorgehen wie in Beispiel 1, mit dem Unterschied, daß eine der 6 Kulturkammern als Testkammer verwendet wird. Die Testkammer enthält als semipermeable Membran eine regenerierte Zellulose mit einem cut off von 5000 Dalton, sie ist durchlässig für niedermolekulare Substanzen, darunter fällt auch das Chemotherapeutikum, nicht jedoch für die makromolekularen Bestandteile des Nährmediums und der Stoffwechselprodukte. Zur Beschickung der Testkammer dient als inertes Füllmedium 2 ml Phosphatpuffer, 0,02 molar mit 0,9% NaCl, eingestellt auf einen pH-Wert von 7,4 und 50 mg Mannan/ml (Molgewicht 80000). Letzteres gleicht den kolloidosmotischen Druck des Nährmediums aus. Nach Beendigung der Inkubation wird im Inhalt der Testkammer die freie ungebundene Ceftriaxon-Konzentration bestimmt, z.B. mit HPLC. Je reichhaltiger das Nährmedium ist und je höher die Eiweißbindung des untersuchten Chemotherapeutikums ist, desto größer ist die Differenz zwischen der analytisch eingewogenen Menge und der ungebundenen frei verfügbaren Menge. Mit dem beschriebenen Verfahren kann die MHK als Konzentration des ungebundenen freien Ceftriaxons unabhängig vom eingesetzten Nährmedium bestimmt werden. Diese Werte können mit denen, der in vivo gemessenen freien ungebundenen Ceftriaxonmengen korreliert werden.

Wie vorstehend erwähnt, betrifft die Erfindung auch die Vorrichtung zur Durchführung des erfindungsgemäßen Verfahrens, die dadurch gekennzeichnet ist, daß sie mehrere Kammern, darunter mindestens eine Kulturkammer für das beimpfte Nährmedium mit jeweils einer oder mehreren für die zu kultivierenden Mikroorganismen undurchlässigen semipermeablen Wand oder Wände und mindestens eine über die semipermeable Wand oder Wände damit in Verbindung stehende Reservoirkammer für das sterile Nährmedium aufweist.

Im folgenden werden bevorzugte Ausführungsformen dieser Vorrichtung beschrieben:

Vorzugsweise weisen die Kammern im unteren Bereich Gaseinlässe auf und im oberen Bereich eine Entnahmeöffnung. Bevorzugt enthalten die Kammern Rührvorrichtungen. Vorzugsweise haben die semipermeable Wand oder Wände einen Porendurchmesser von 0,5 nm (entsprechend einer nominalen Molekulargewichtsgrenze (NMGG) von 500 Dalton) bis 5 /um, und besonders bevorzugt von 5 nm (entsprechend einer NMGG von 5000 Dalton) bis 0,2 /um. Bevorzugt ist das Verhältnis Gesamtvolumen der Kulturkammern zu Volumen der Reservoirkammer 1:1 bis 1:2000 besonders bevorzugt 1:5 bis 1:70 und insbesondere 1:5 bis 1:30.

Bevorzugt weist die Vorrichtung eine Kulturkammer pro Reservoirkammer auf und vorzugsweise ragt die Kulturkammer von oben in die Reservoirkammer hinein, wobei der Boden der Kulturkammer und/oder die Seitenwände zumindest teilweise als semipermeable Wand ausgebildet sind. Bei dieser Ausführungsform ist vorzugsweise eine Hilfe zur Bestimmung des Wachstumsverhaltens der Mikroorganismen und/oder Zellen in die Kulturkammer eingebracht, bevorzugt eine optische Hilfe zur Bestimmung der Trübung des Kulturmediums, welche vorzugsweise eine Verspiegelung oder Verschwärzung darstellt. Vorzugsweise sind dabei in den Kammern Rührkörper vorhanden.

Die erfindungsgemäße Vorrichtung weist bevorzugt eine Testkammer für das inerte Medium auf, die über eine oder mehrere für das Chemotherapeutikum durchlässige semipermeable Wände mit einer der anderen Kammern in Verbindung steht, wobei die semipermeable Wand oder Wände der Testkammer vorzugsweise eine Durchlässigkeit von 500 bis 100000 Dalton, insbesondere von 1000 bis 5000 Dalton aufweisen. Bei der Ausführungsform, die eine Hilfe zur Bestimmung des Wachstumsverhaltens der Mikroorganismen und/oder Zellen in der Kulturkammer enthält, beträgt vorzugsweise das Verhältnis von Testkammervolumen zu Kulturkammervolumen 1:1 bis 1:50 und vorzugsweise weist die Vorrichtung eine Palette von Kulturkammern und Reservoirkammer-Einheiten in Form einer Petrischale auf, wobei die Palette von Kultur-/Reservoirkammer-Einheiten bevorzugt maschinenkompatibel z.B. rechteckig, gestaltet ist.

0200226

PATENTANSPRÜCHE

1.) Verfahren zur Bestimmung der Wirkung von Chemotherapeutika auf das Wachstum von Mikroorganismen und/oder
Zellen, wobei die zu untersuchenden Mikroorganismen und/oder
Zellen in ein Chemotherapeutikum enthaltendes Nährmedium
inokuliert und darin bebrütet werden und ihr Wachstumsverhalten bestimmt wird, d a d u r c h g e k e n n -
z e i c h n e t, daß das beimpfte Nährmedium mit einem
sterilen, das Chemotherapeutikum ebenfalls enthaltenden
Nährmedium derart in Verbindung gebracht wird, daß Nährstoffe,
Chemotherapeutikum und, falls gewünscht, Stoffwechselprodukte der Mikroorganismen und/oder Zellen, nicht jedoch
die Mikroorganismen selbst, zwischen den Nährmedien permeiren.

2.) Verfahren nach Anspruch 1, d a d u r c h g e k e n n -
z e i c h n e t, daß das beimpfte oder das sterile Nährmedium mit einem inerten Medium derart in Verbindung gebracht wird, daß der freie, ungebundene Anteil des Chemotherapeutikums aus den Nährmedien in das inerte Medium
permeirt.

3.) Verfahren nach Anspruch 2, dadurch gekennzeichnet, daß
in inerten, wässrigen Medien Osmolarität und kolloidosmotischer Druck auf in den Nährmedien herrschende Werte
eingestellt werden.

4.) Verfahren nach Anspruch 2, dadurch gekennzeichnet, daß
auch nichtwässrige Medien für die Aufnahme der in den
Nährmedien ungebundenen, frei diffundierenden Moleküle
des Chemotherapeutikums eingesetzt werden.

5.) Verfahren nach einem der Ansprüche 1 bis 4 dadurch gekennzeichnet, daß durch Einleiten von Gasen für das Wachstum von Mikroorganismen und/oder Zellen gewünschte Gaspartialdrücke eingestellt werden.

6.) Verfahren nach Anspruch 5, dadurch gekennzeichnet, daß über Gaspartialdrücke der pH-Wert eingestellt und gesteuert wird.

7.) Verfahren nach einem der Ansprüche 1 bis 6, dadurch gekennzeichnet, daß die Medien, jedes für sich, durchmischt werden.

8.) Verfahren nach Anspruch 7, dadurch gekennzeichnet, daß die Medien durch Einleiten von Gasen durchmischt werden.

9.) Vorrichtung zur Durchführung des Verfahrens nach einem der Ansprüche 1 bis 8, dadurch gekennzeichnet, daß sie mehrere Kammern, darunter mindestens 1 Kulturkammer für das beimpfte Nährmedium mit jeweils einer oder mehreren für die zu kultivierenden Mikroorganismen undurchlässigen semipermeablen Wand oder Wände und mindestens eine über die semipermeable Wand oder Wände damit in Verbindung stehende Reservoirkammer für das sterile Nährmedium aufweist.

10.) Verwendung einer Vorrichtung mit mehreren Kammern, darunter mindestens 1 Kulturkammer für das beimpfte Nährmedium mit jeweils einer oder mehreren für die zu kultivierenden Mikroorganismen undurchlässigen semipermeablen Wand oder Wänden und mindestens einer über die semipermeable Wand oder Wände damit in Verbindungen stehenden Reservoirkammer für das sterile Nährmedium zur Durchführung des Verfahrens nach einem der Ansprüche 1 bis 8.

# DREIKAMMERSYSTEM

0200226

1/6

Fig. 1

Öffungen  3

Reservoirkammer R

Kulturkammer  K

Membran  1

Testkammer  T

Membran  2

Gaseinlässe  4

Fig. 2

TESTKAMMER FÜR WISSENSCHAFTLICHE ZWECKE

Entnahmebohrung 6

Brennerbohrung 7

Zif. 8

Breitere Platte 9

Zif. 5

Kolbenstab

Kultur oder Testkammer K bzw. T

Reservoirkammer R

Fig. 2a

TESTKAMMER FÜR WISSENSCHAFTLICHE ZWECKE

Entnahmebohrung 6

Edelstahlstäbe

Begasungsbohrung 7

Breitere Platte 9

Zif. 8

Zif. 5

Kultur oder Testkammer K bzw. T

Fig. 3

Eine Kulturkammer und ein Membranhalter aus dem in Fig. 2
dargestellten Mehrkammersystem.

Membran   10

Austauschkammer und Membranhalter   9     Kultur oder Testkammer K bzw. T

Fig. 3a

Vorsprung 12

Austauschkammer und Membranhalter 9

Kultur oder Testkammer K bzw. T

Fig. 4

Füllöffnung F

Kulturkammer K

Kulturkammer K

Reservoirkammer R

Aufsicht (Maßstab ca.1:1)

Fig. 5

8mm

5mm

Kulturkammer k

Reservoirkammer R

Semipermeable Wand 10

Seitenansicht (Maßstabfrei)

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | THE JOURNAL OF ANTIBIOTICS, Band 32, Nr. 11, November 1979, Seiten 1168-1173; G. MASUDA et al.: "Comparative bactericidal activities of beta-lactam antibiotics determined in agar and broth media" * Insgesamt * | 1,9,10 | C 12 Q 1/18 C 12 M 1/12 // C 12 M 1/04 (C 12 Q 1/18 C 12 R 1:36 ) |
| Y,P | EP-A-0 155 237 (MBR BIO REACTOR AG) * Insgesamt * | 1,9,10 | |
| A | | 2,5 | |
| Y | WO-A-8 403 047 (BROWN UNIVERSITY RESEARCH FOUNDATION) * Zusammenfassung; Seite 10, Zeile 22 - Seite 12, Zeile 33; Ansprüche; Figuren 1,2a * | 1,9,10 | |
| A | FR-A-1 220 345 (TECHNICON INSTRUMENTS CORP.) * Insgesamt * --- -/- | 1,9,10 | RECHERCHIERTE SACHGEBIETE (Int. Cl.4) C 12 Q C 12 M |

Der vorliegende Recherchenbericht wurde für alle Patentansprüche erstellt

| Recherchenort | Abschlußdatum der Recherche | Prüfer |
|---|---|---|
| DEN HAAG | 14-08-1986 | HITCHEN C.E. |

**Europäisches Patentamt**

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (int Cl 4) |
|---|---|---|---|
| A | FR-A-2 140 576 (MAX-PLANCK GESELLSCHAFT ZUR FÖRDERUNG DER WISSENSCHAFTEN E.V.) <br> * Seite 2, Zeile 5 - Seite 3, Zeile 1; Seite 4, Zeilen 17-27; Seite 5, Zeile 27 - Seite 6, Zeile 27 * | 1,5,6 | |

-----

RECHERCHIERTE SACHGEBIETE (Int Cl 4)

Der vorliegende Recherchenbericht wurde für alle Patentansprüche erstellt

| Recherchenort | Abschlußdatum der Recherche | Prüfer |
|---|---|---|
| DEN HAAG | 14-08-1986 | HITCHEN C.E. |